# FASCICULE DE BREVET EUROPEEN

(11) **EP 0 524 895 B1**
(45) Date de publication et mention de la délivrance du brevet: **13.08.1997**
(21) Numéro de dépôt: 92420216.1
(22) Date de dépôt: 24.06.1992
(51) Int. Cl.: C07C 255/08, C07C 253/24

(54) **Procédé d'ammoxydation d'hydrocarbures saturés**
Verfahren zur Ammoxydation von gesättigten Kohlenwasserstoffen
Process for the ammoxidation of saturated hydrocarbons

(30) Priorité: 04.07.1991 FR 9108643
(43) Date de publication de la demande: 27.01.1993
(73) Titulaire: RHONE-POULENC FIBER & RESIN INTERMEDIATES, 92405 Courbevoie (FR)
(72) Inventeur: Blanchard, Gilbert, F-60330 Le Plessis Belleville (FR); Bordes, Elisabeth, F-95470 Vemars (FR); Ferré, Gilbert, F-93190 Livry Gargan (FR)
(74) Mandataire: Vignally, Noel

(56) Documents cités:
- FR-A- 1 462 595
- FR-A- 1 488 234
- FR-A- 2 027 238
- FR-A- 2 072 399
- FR-A- 2 119 492
- FR-A- 2 119 493

## Description

La présente invention concerne un procédé d'ammoxydation d'hydrocarbures saturés c'est-à-dire la conversion d'alcanes en un mélange renfermant des nitriles α,β-insaturés.

Il est bien connu de l'homme de l'art que de très nombreuses propositions ont été formulées en ce qui concerne l'ammoxydation des oléfines et, en particulier celle du propylène. Toutefois, bien que les hydrocarbures saturés, plus largement disponibles, soient des matières premières plus intéressantes au plan économique, il est également bien connu qu'ils ne présentent pas une réactivité comparable dans ce type de réaction pour former notamment des nitriles α,β-insaturés.

L'une des difficultés rencontrées dans l'ammoxydation des hydrocarbures saturés réside dans la nécessité de pouvoir disposer de catalyseurs susceptibles de déshydrogéner l'hydrocarbure saturé dans des conditions minimisant ou supprimant la combustion de l'ammoniac et/ou celle de l'hydrocarbure tout en assurant une sélectivité raisonnable soit en nitrile α,β-insaturé (produit visé) par exemple en acrylonitrile au départ du propane, soit en produits valorisables (nitrile précité et oléfine), par exemple, en acrylonitrile et propylène au départ du propane.

Il a déjà été proposé dans le brevet américain n° 3 365 482 et le brevet français FR-A-1 462 595 d'ammoxyder notamment l'isobutane en méthacrylonitrile sur un catalyseur à base de molybdène déposé sur de l'éta-alumine, dopée par de l'antimoine à 508°C, au départ d'un mélange gazeux renfermant de l'isobutane, de l'air, de l'ammoniac et de la vapeur d'eau (1,0/4,5/1,0/12,5), la sélectivité en méthacrylonitrile atteint 49 % pour un taux de conversion de l'isobutane de 22 %.

Au départ d'un mélange gazeux propane/air/ammoniac/vapeur d'eau (1,0/4,7/0,67/12,8), avec le même catalyseur et à 550°C, la sélectivité en acrylonitrile tombe à 15 % pour un taux de transformation du propane de 29 %.

Le brevet FR-A-1 488 234 décrit de manière plus détaillée les catalyseurs mis en oeuvre dans les brevets cités précédemment.

Dans le brevet français 2 027 238 (correspondant pour partie au brevet américain n° 3 670 009) il est proposé un procédé d'ammoxydation en phase vapeur d'hydrocarbures saturés à une température supérieure à 500°C sur un catalyseur solide qui peut notamment être constitué d'oxyde d'étain, d'oxyde de bore, d'oxyde de molybdène et d'oxyde de silicium. Ainsi, dans l'exemple IX du tableau pages 12-13, la sélectivité en acrylonitrile atteint 35 %, à 32 % de conversion du propane, mais avec des conditions opératoires plaçant le mélange réactionnel propane/ammoniac/air (1/1,2/12) dans le domaine d'explosivité.

Dans le brevet français 2 072 334 (correspondant au brevet britannique n° 1 336 135) il est préconisé un procédé d'ammoxydation catalytique d'alcanes en phase vapeur, à une température inférieure à 500°C avec une forte concentration d'alcane dans le mélange gazeux alimenté, sur un catalyseur solide qui peut notamment être constitué d'oxyde d'étain et d'oxyde de molybdène (90/10 en poids) ; toutefois de meilleures performances sont obtenues avec des catalyseurs constitués d'oxyde d'antimoine et d'oxyde de vanadium.

Dans le brevet français 2 072 399 il est également proposé un procédé d'ammoxydation catalytique d'alcane, en phase vapeur, avec une forte concentration d'alcane dans le mélange gazeux alimenté, sur un catalyseur solide qui peut notamment être un mélange binaire d'oxydes comportant de l'oxyde de molybdène.

Sont plus particulièrement à signaler les couples suivants :
(Mo, Sb) (Mo, Sn) (Mo, V) (Mo, Ti) (Mo, Bi).

Toutefois aucun de ces couples n'offre de performances meilleures que celles obtenues avec des couples ne renfermant pas de molybdène. Les rendements en acrylonitrile obtenus sont très faibles ; au mieux, 1,7 % du propane est converti en acrylonitrile à 570°C sur un catalyseur à base d'oxydes d'étain et de titane.

Dans le brevet français n° 2 119 492 (correspondant au brevet américain n° 3 746 737 et au brevet britannique n° 1 337 759) il est proposé d'utiliser une composition binaire à base d'oxydes de molybdène et de cérium. Toutefois, les performances du couple (Mo, Ce) paraissent insuffisantes en l'absence d'halogène ou de composé halogéné.

Il est en outre préconisé d'ajouter à cette composition binaire (Mo, Ce) un troisième élément choisi parmi le tellure et le bismuth (cf. également US 3 833 638). Là encore, les performances du système catalytique paraissent insuffisantes en l'absence d'halogène ou de composé halogéné. Au surplus, on notera qu'en présence de CH₃Br, la sélectivité en acrylonitrile atteint 67 % à 98 % de conversion du propane, mais dans des conditions opératoires plaçant le mélange réactionnel propane/ammoniac/air (1/1, 2/12) dans le domaine d'explosivité.

Dans le brevet français n° 2 119 493 il a également été proposé de réaliser l'ammoxydation d'alcanes en phase vapeur sur un catalyseur solide renfermant des oxydes de bismuth et de molybdène et le cas échéant du phosphore et de la silice.

Là encore les performances du système catalytique paraissent insuffisantes en l'absence d'halogène ou de composé halogéné et le mélange réactionnel est dans le domaine d'explosivité.

Face à ces nombreuses insuffisances, divers travaux parallèles ou subséquents ont porté sur l'utilisation de catalyseurs solides à base de vanadium et/ou d'antimoine.

Dans "Chemistry letters 1989 (pp 2173-2176)" des auteurs ont testés dans l'ammoxydation du propane en phase vapeur des oxydes métalliques multicomposants renfermant du molybdène et du bismuth et présentant une structure du type de celle de la scheelite. Il apparaît que, malgré les températures relativement modérées mises en oeuvre, la proportion de produits de combustion (CO, CO₂) est très élevée dans tous les cas (au moins 15 %) et que certaines compositions catalytiques testées sont très peu actives vis-à-vis de la réaction souhaitée malgré leur mise en oeuvre dans des conditions se situant dans le domaine d'explosivité ou très proches dudit domaine.

La présence de composé halogéné est susceptible d'induire une corrosion de l'appareillage et n'est donc pas souhaitable dans un procédé industriel.

Il est par ailleurs manifeste que la coproduction de grandes quantités de CO et de CO₂ est indésirable à l'échelle industrielle.

Au surplus, l'utilisation de mélanges réactionnels se situant dans le domaine d'explosivité est d'autant moins souhaitable à l'échelle industrielle que l'on met en oeuvre le procédé en lit fixe.

Il apparaît donc qu'il serait hautement souhaitable de disposer d'un procédé d'ammoxydation d'alcanes permettant d'obtenir avec une sélectivité appréciable un mélange de produits valorisables renfermant un nitrile α,β-insaturé en particulier de l'acrylonitrile, tout en diminuant les pertes de matière première par suite de la formation d'oxydes de carbone.

Il serait également hautement souhaitable de disposer d'un tel procédé dans lequel le catalyseur solide serait relativement simple à préparer et actif en l'absence de promoteur halogéné et pour des mélanges de gaz ne se situant pas nécessairement dans le domaine d'explosivité.

La présente invention a donc pour objet un procédé d'ammoxydation d'alcanes en phase vapeur en présence d'un catalyseur solide dont la phase active renferme du molybdène et de l'oxygène caractérisé en ce que la dite phase active est un oxyde mixte à base de molybdène ou un orthomolybdate qui renferme également au moins un élément choisi dans le groupe constitué par les métaux alcalino-terreux, Mn, Fe, U, La, Co.

Selon la présente invention des hydrocarbures saturés acycliques ayant de 3 à 12 atomes de carbone par molécule sont mis à réagir en phase vapeur avec de l'ammoniac et de l'oxygène en présence d'un catalyseur dont la phase active vient d'être précisée.

Bien entendu, dans le cadre du présent procédé, on peut utiliser des gaz diluants inertes dans les conditions réactionnelles tels que l'hélium, l'azote et l'argon. De même, de la vapeur d'eau peut être ajoutée au mélange gazeux réactionnel dans de larges limites. Ainsi, le gaz réactif (hydrocarbure saturé, ammoniac, oxygène) peut être dilué par un diluant inerte et/ou par de la vapeur d'eau. Dans cet ensemble la teneur en vapeur d'eau peut varier dans de larges limites en particulier de 0 à 50 % et, de préférence entre 3 et 30 %. Pour une bonne mise en oeuvre du procédé selon l'invention la teneur en gaz réactif sera d'au moins 3 % et de préférence d'au moins 20 %.

Au sein du gaz réactif les teneurs respectives en hydrocarbure saturé, ammoniac et oxygène peuvent varier dans de larges limites.

La teneur en hydrocarbure saturé dans le gaz réactif est de préférence comprise entre 5 et 70 %. Celle en ammoniac est de préférence comprise entre 3 et 50 % et celle en oxygène est de préférence comprise entre 3 et 45 %.

Pour une bonne mise en oeuvre du procédé selon l'invention la composition du mélange réactif sera en dehors du domaine d'explosivité. S'agissant de l'ammoxydation du propane en l'absence de diluant inerte, la composition (propane, oxygène, ammoniac) sera avantageusement choisie à l'intérieur du quadrilatère ABDE figurant au sein du diagramme ternaire ABC représenté sur la figure unique annexée.

Sur ce diagramme ternaire le segment AB représente la teneur en ammoniac de 100 % à 0 % ; le segment BC représente la teneur en propane de 100 à 0 % ; le segment CA représente la teneur en oxygène de 100 à 0 %. Le point D, situé à l'intérieur du segment BC, correspond à une teneur en propane de 45 % ; dans le binaire (propane-O₂) ; le point E situé à l'intérieur du segment AC, correspond à une teneur en ammoniac de 79 % dans le binaire (NH₃)-O₂) %.

Le segment DE délimite le diagramme ternaire en deux parties un triangle CDE à l'intérieur duquel se situe la zone d'explosivité (déterminée sous 1 bar et à 25°C) et un quadrilatère ABDE à l'intérieur duquel la composition du mélange gazeux réactif sera avantageusement choisie.

S'agissant de l'ammoxydation du propane en présence de gaz diluant(s) inerte(s) et/ou de vapeur d'eau, il conviendra de déterminer la composition du mélange ternaire (propane, oxygène et ammoniac) pour la situer sur le diagramme précité, lorsque le gaz diluant et/ou la vapeur d'eau est en faible proportion.

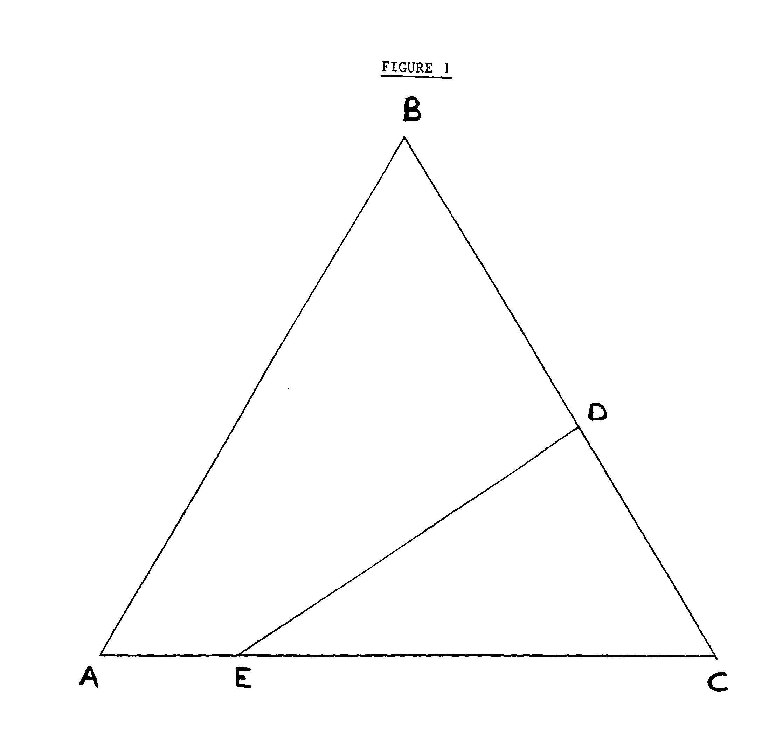

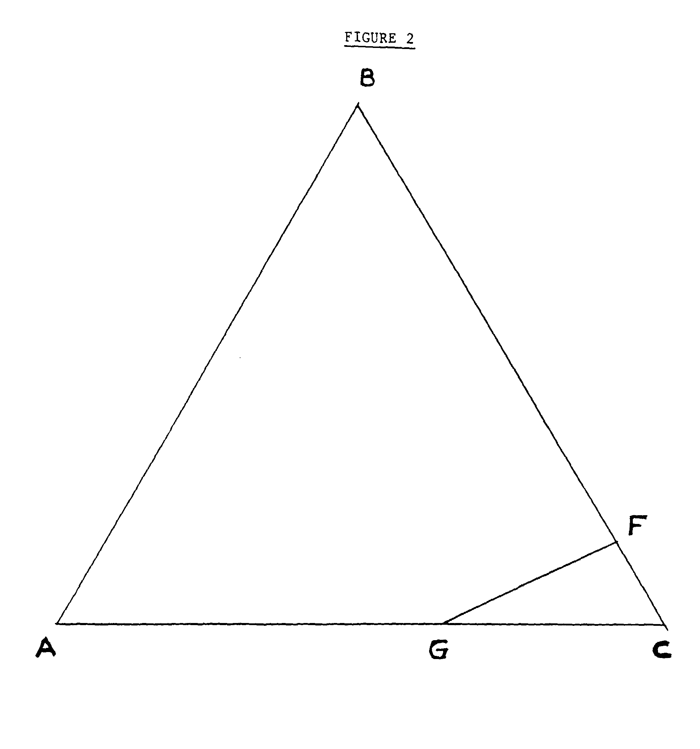

S'agissant de l'ammoxydation du propane au moyen de l'air comme source d'oxygène, la composition (propane, air et ammoniac) sera avantageusement choisie à l'intérieur du quadrilatère ABFG figurant au sein du diagramme ABC représenté sur la figure 2 annexée.

Sur ce second diagramme le segment AB représente la teneur en ammoniac de 100 % à 0 % ; le segment BC représente la teneur en propane de 100 à 0 % : le segment CA représente la teneur en air de 100 à 0 %. Le point F, situé à l'intérieur du segment BC, correspond à une teneur en propane de 16 % dans le binaire (propane-air) ; le point G situé à l'intérieur du segment AC, correspond à une teneur en ammoniac de 35 % dans le binaire (ammoniac-air).

Le segment FG délimite le diagramme ternaire en deux parties : un triangle CFG à l'intérieur duquel se situe la zone d'explosivité (déterminée sous 1 bar à 550°C) et un quadrilatère ABFG à l'intérieur duquel la composition du mélange gazeux réactif sera avantageusement choisie.

Ce second diagramme sera utilisé dans le cas où le mélange oxygène-gaz diluant correspond à une teneur en oxygène équivalente à celle de l'air (≃ 21 % d'oxygène) ou dans le cas où ce mélange est en défaut d'oxygène, par rapport à l'air.

Au départ de propane on obtiendra un mélange renfermant essentiellement du propylène et de l'acrylonitrile. L'acrylonitrile est un intermédiaire industriellement produit sur une large échelle, le propylène est une matière première traditionnellement utilisée pour produire de l'acrylonitrile, et divers autres produits intermédiaires bien connus de l'homme de l'art.

Au départ d'isobutane on obtiendra un mélange renfermant du méthacrylonitrile et de l'iso-butène ou des n-butènes.

Le procédé selon l'invention convient plus particulièrement à l'ammoxydation du propane.

Si l'hydrocarbure saturé mis en oeuvre peut être de qualité technique, il ne renfermera pas de quantités notables de composés à insaturation éthylénique. Ainsi le propane engagé ne renfermera de propylène qu'à l'état de traces.

Le procédé selon l'invention et réalisé sous forme de réaction en phase vapeur. En conséquence n'importe quel dispositif convenant à la réalisation des réactions d'ammoxydation ou d'oxydation en phase vapeur peut être utilisé. Le procédé peut être conduit en continu ou en discontinu et il peut comprendre l'utilisation d'un lit fixe ou d'un lit fluidisé.

La température de réaction est en général comprise entre 350 et 550°C et, de préférence entre 420 et 510°C.

La pression totale du mélange réactionnel peut être supérieure ou égale à la pression atmosphérique. Elle est généralement comprise entre 1 et 6 bar et, de préférence entre 1 et 4 bar.

Le débit gazeux est fixé de telle sorte que la vitesse volumique horaire soit comprise entre 100 et 36000 h⁻¹ et, de préférence entre 200 et 20000 h⁻1.

Bien entendu, l'homme de l'art sera à même de trouver un compromis entre la température, le débit gazeux, la nature précise du catalyseur mise en oeuvre et les divers autres paramètres de la réaction compte-tenu de ses objectifs de production.

Dans le procédé selon la présente invention on met en oeuvre un catalyseur solide dont la phase active renferme du molybdène et de l'oxygène, ladite phase active est un oxyde mixte à base de molybdène ou un orthomolybdate renfermant également au moins un élément choisi dans le groupe constitué par les métaux alcalino-terreux, Mn, Fe, U, La, Co.

Les phases actives, l'un des constituants du catalyseur mis en oeuvre dans le cadre de la présente invention sont des oxydes mixtes à base de molybdène qui renferment, de préférence au maximum 2 éléments choisis dans le groupe défini ci-avant. De bons résultats peuvent être obtenus dans le cadre du présent procédé par la mise en oeuvre d'une telle phase active renfermant au moins un élément choisi dans le groupe constitué par les métaux alcalino-terreux, le manganèse, le fer, le cobalt, l'uranium et le lanthane.

Les phases actives en cause sont, de préférence des molybdates. Les phases actives préférées sont les molybdates de manganèse, d'uranyle, de cobalt ou de fer.

Si dans les phases actives en cause la proportion d'élément(s) M choisi(s) dans les groupes précités peut varier dans de larges limites, le minimum sera, de préférence, dicté par la stoechiométrie de formation de l'orthomolybdate correspondant. En diminuant la proportion d'élément(s) M on obtiendra, dans certain cas et selon la nature de M, un mélange du molybdate correspondant et d'un oxyde de molybdène.

Ainsi, par exemple, un large excès de molybdène par rapport au fer (Mo/Fe = 10) conduit à la formation d'un mélange de Fe₂(MoO₄)₃ et de MoO₃ dont les performances dans le procédé en cause paraissent moins bonnes que celles obtenues avec Fe₂(MoO₄)₃.

Les phases actives en cause peuvent être mises en oeuvre dans le cadre du procédé selon l'invention sous forme massique ou à l'état particulaire. Ces phases peuvent être utilisées sous forme de poudres, billes, extrudées ou concassées, par exemple.

Elles peuvent être également déposées sur support inerte ou l'enrober. La nature du support n'est pas critique dès lors qu'il est chimiquement inerte vis-à-vis des réactifs dans les conditions réactionnelles choisies. A titre d'exemples de supports susceptibles de convenir à la préparation de catalyseurs utilisables dans le cadre du procédé selon l'invention, on peut citer : la silice, l'alumine, la silice-alumine, l'argile frittée, le carborandum, la magnésie, le silicate de magnésium, la terre de diatomée. Ce support est de préférence non poreux et peut être notamment, à base d'oxyde réfractaire sous forme particulaire, le support le plus couramment employé étant à base d'argile. Ce support peut par exemple être constitué de billes d'argile, inertes, pleines, solides et rugueuses, de diamètre compris entre 0,5 et 6 mm. La valeur précise du diamètre des billes pourra être choisie par l'homme de l'art en fonction de la perte de charge admissible dans le réacteur. Le support peut également être rendu non poreux par émaillage.

Le support peut également être un substrat céramique, ledit substrat étant de préférence sous forme d'une structure inerte et rigide de type monolithique comprenant des canaux ou conduits. De tels supports sont bien connus de l'homme de l'art et ont été largement décrits dans la littérature. Les substrats en matière céramiques utilisés sont notamment ceux comportant comme matière principale la cordiérite, l'alumine, la mullite, la porcelaine, les carbures de bore ou de silicium.

Lorsqu'on fait appel à un catalyseur enrobé, la quantité de phase active qui peut varier dans de larges limites, est en pratique comprise entre 5 et 35 % et, de préférence entre 10 et 15 % en poids par rapport à l'ensemble (support + phase active).

La préparation des catalyseurs mis en oeuvre dans le procédé selon l'invention peut être réalisée de diverses manières connues en soi, tel le mélange de sels convenables des constituants élémentaires dans l'eau ou dans un autre solvant suivi de l'évaporation jusqu'à siccité, ou de la précipitation par ajout d'une base telle l'ammoniaque ou d'un acide tel l'acide chlorhydrique, ou l'atomisation d'une suspension obtenue après mélange des sels convenables.

Les sels convenables les plus couramment employés sont solubles dans l'eau et contiennent des anions et des cations qui peuvent être décomposés par la chaleur lors des étapes ultérieures. Ce sont, par exemple, l'heptamolybdate d'ammonium pour le molybdène, les nitrates ou chlorures de métaux alcalino-terreux, de manganèse, de fer, de cobalt, d'uranium, de lanthane ... pour les métaux.

Une fois le mélange des sels réalisé, un précurseur peut être obtenu par la méthode dite d'évaporation. L'eau de la suspension obtenue est évaporée en chauffant entre 20 et 100°C sous agitation pendant le temps nécessaire à l'obtention d'une pâte non coulante. L'agitation et le chauffage sont alors arrêtés.

La pâte ainsi obtenue, étalée sur une épaisseur de 2 cm environ, est séchée sous air à 120°C environ pendant 15 h environ. Le précurseur ainsi obtenu peut ensuite être broyé et calciné entre 200 et 1000°C, de préférence ente 400 et 600°C, pendant au moins 30 mn, de préférence au moins une heure. La calcination peut s'effectuer en montant progressivement la température, 100 à 200°C par heure par exemple, en raison, notamment, des risques liés à la décomposition exothermique du nitrate d'ammonium vers 230°C. La phase active ainsi obtenue après refroidissement peut ensuite être broyée pour que sa granulométrie n'excède pas 400 µm environ.

Le précurseur peut aussi être obtenu selon une variante comprenant la précipitation avec addition, par exemple, d'ammoniaque ou d'acide chlorhydrique en fin de mélange des sels pour stabiliser le pH vers 7 environ. Il est préférable de chauffer la suspension entre 20 et 100°C pendant environ une heure pour parfaire la précipitation des espèces.

Puis la suspension est filtrée et lavée. Le gâteau de filtration est ensuite étalé, puis séché, broyé et calciné selon les conditions décrites ci-avant dans le cadre de la méthode d'évaporation, pour donner la phase active.

Certains catalyseurs, utiles pour une mise en oeuvre du procédé en lit fixe, peuvent être obtenus par enrobage de manière connue en soi, des phases actives, intermédiaires ou finies, broyées. Cette méthode classique consiste à déposer autour de billes inertes mais rugueuses une couche de phase active intermédiaire ou finie.

Une fois les billes recouvertes de la quantité voulue de la phase active, elles sont séchées par de l'air chaud, entre 70 et 150°C pendant au moins 30 minutes, puis introduites dans un four pour être calcinées entre 300 et 600°C de préférence entre 450 et 500°C, pendant au moins 3 heures.

Certains catalyseurs utiles pour une mise en oeuvre du procédé selon l'invention en lit mobile ou lit fluidisé peuvent être obtenus par la technique en soi connue du séchage par atomisation en atmosphère, de préférence, non réductrice. Par une telle opération, le cas échéant suivie d'une calcination à une température de l'ordre de 400 à 1100°C, on obtient des poudres de forme sphérique de diamètre compris entre 5 et 700 µm. Des poudres constituées pour au moins 80 % en poids de particules dont la dimension est comprise entre 5 et 100 µm sont préférées dans la cadre d'une utilisation en lit fluidisé.

Les produits de la réaction peuvent être récupérés dans les gaz effluents par tout moyen approprié. Par exemple les gaz effluents peuvent passer dans un condenseur contenant de l'acide sulfurique dilué pour neutraliser l'ammoniac non converti. Les gaz peuvent ensuite passer sur une colonne absorbante réfrigérée pour condenser l'acrylonitrile, l'acétonitrile et l'acide cyanhydrique, les vapeurs non condensées contenant principalement du propane non converti du propylène, des hydrocarbures légers et le cas échéant du CO₂. On peut ensuite séparer l'acrylonitrile et l'acide cyanhydrique de l'acétonitrile par distillation, puis distiller à son tour le mélange acrylonitrile-acide cyanhydrique récupéré pour séparer l'acrylonitrile de l'acide cyanhydrique.

Les exemples ci-après illustrent la présente invention.

### EXEMPLE 1 : PREPARATION D'UN CATALYSEUR A BASE DE MOLYBDATE DE MAGNESIUM :

On prépare une solution (a) d'heptamolybdate d'ammonium par dissolution de 52,98 g de (NH₄)₆Mo₇O₂₄,4H₂O (commercialisé par la Société MERCK) dans 200 cm3 d'eau permutée (pH de la solution obtenue : 5-6), et une solution (b) de nitrate de magnésium par dissolution de 79,3 g de Mg(NO₃)₂, 6H₂O 97 % (commercialisé par la Société PROLABO) dans 200 cm3 d'eau permutée. La solution (b) est ajoutée à la solution (a) dans un réacteur agité (pH=5). On chauffe le mélange sur plaque chauffante et la pâte obtenue est séchée à 120°C pendant 15 h environ ; le produit obtenu est ensuite broyé dans un mortier et calciné sous air à 500°C pendant 4 h.

Le produit (I) ainsi préparé a pour composition MgMoO₄, identifiée par diffraction des rayons X, et présente une surface spécifique mesurée selon la méthode B.E.T. de 5m²g⁻¹.

20 g du produit (I) sont saupoudrés lentement sur 123 g de support inerte composé de billes d'argile de diamètre moyen 4,8 mm, préalablement placées dans un drageoir en rotation et humidifiées par du glucose en solution aqueuse à 10 %. Dès que les billes sont sèches à l'extérieur, on pulvérise une petite quantité de la solution de glucose. Puis on saupoudre à nouveau le produit (I) sur les billes. On poursuit ces opérations alternativement jusqu'à enrobage de la totalité du produit (I). On sèche ensuite à 120°C pendant 2 h et calcine à 480°C pendant 6 h.

Le catalyseur (A) ainsi obtenu est constitué de 12 % en poids de MgMoO₄ déposés sur billes d'argile.

### EXEMPLE 2 - PREPARATION D'UN CATALYSEUR A BASE DE MOLYBDATE DE MANGANESE -

On prépare une phase active de composition MnMoO₄ selon le protocole opératoire décrit par U. OZRAN, R.C. GILL & M.R. SMITH, J. Catal. 116, 171 - 183 (1989).

On prépare une solution (a) d'heptamolybdate d'ammonium par dissolution de 70,64 gde (NH₄)₆Mo₇O₂₄, 4H₂O (commercialisé par la Société MERCK) dans 200 cm3 d'eau permutée, et une solution (b) de chlorure de manganèse par dissolution de 79,16 g de MnCl₂, 4H₂O (commercialisé par la Société PROLABO) dans 400 cm3 d'eau permutée. La solution (a) est ajoutée goutte à goutte à la solution (b) dans un réacteur fortement agité. On chauffe à 80°C et maintient le pH à 6 par ajout d'acide chlorhydrique dilué ou d'ammoniaque (commercialisés par la Société PROLABO) pendant 3 h. On filtre à chaud sur verre fritté et lave par 1l d'eau permutée. Le produit obtenu est ensuite séché à 120°C pendant 15 h environ, broyé dans un mortier et calciné sous air à 500°C pendant 4 h.

Le produit (I) ainsi préparé a pour composition MnMoO₄, identifiée par diffraction des rayons X, et présente une surface spécifique mesurée selon la méthode B.E.T. de 5m2g⁻¹.

20 g du produit (I) sont saupoudrés lentement sur 123 g de support inerte composé de billes d'argile de diamètre moyen 4,8 mm, préalablement placées dans un drageoir en rotation et humidifiées par du glucose en solution aqueuse à 10 %. Dès que les billes sont sèches à l'extérieur, on pulvérise une petite quantité de la solution de glucose. Puis on saupoudre à nouveau le produit (I) sur les billes. On poursuit ces opérations alternativement jusqu'à enrobage de la totalité du produit (I). On sèche ensuite à 120°C pendant 2 h et calcine à 480°C pendant 6 h.

Le catalyseur (B) ainsi obtenu est constitué de 11 % en poids de MnMoO₄ déposés sur billes d'argile.

### EXEMPLE 3 - PREPARATION D'UN AUTRE CATALYSEUR A BASE DE MOLYBDATE DE MANGANESE -

On prépare 20 g de produit (I) de composition MnMoO₄ tel que décrit dans l'exemple précédent. Ce produit est ensuite compressé sous une pression de 20 t. On obtient ainsi des pastilles de 3 cm de diamètre et environ 0,5 cm d'apaisseur. Ces pastilles sont ensuite concassées en éclats de granulométrie comprise entre 0.3 et 0.8 cm, constituant le catalyseur (C).

### EXEMPLE 4 - PREPARATION D'UN CATALYSEUR A BASE DE MOLYBDATE D'URANYLE :

On prépare une phase active de composition UO₂MoO₄ elon le protocole opératoire décrit par E. BORDES, Thèse d'Etat, COMPIEGNE (1979).

On prépare une solution (a) d'heptamolybdate d'ammonium par dissolution de 35,32 g de (NH₄)₆Mo₇O₂₄,4H₂O (commercialisé par la Société MERCK) dans 200 cm3 d'eau permutée, et une solution (b) de nitrate d'uranyle par dissolution de 100,43 g de UO₂(NO₃)₂, 6H₂O (commercialisé par la Société PROLABO) dans 200 cm3 d'eau permutée. La solution (b) est ajoutée à la solution (a) dans un réacteur agité. On ajoute 100 cm3 d'eau permutée, on chauffe sur plaque chauffante et la pâte obtenue est séchée à 120°C pendant 15 h environ ; le produit obtenu est ensuite broyé dans un mortier et calciné sous air à 550°C pendant 12h.

Le produit (I) ainsi préparé a pour composition UO₂MoO₄, identifiée par diffraction des rayons X, et présente une surface spécifique mesurée selon la méthode B.E.T. de 2,7 m2g⁻¹.

20 g du produit (I) sont saupoudrés lentement sur 123 g de support inerte composé de billes d'argile de diamètre moyen 4,8 mm, préalablement placées dans un drageoir en rotation et humidifiées par du glucose en solution aqueuse à 10 %. Dès que les billes sont sèches à l'extérieur, on pulvérise une petite quantité de la solution de glucose. Puis on saupoudre à nouveau le produit (I) sur les billes. On poursuit ces opérations alternativement jusqu'à enrobage de la totalité du produit (I). On sèche ensuite à 120°C pendant 2 h et calcine à 480°C pendant 6 h.

Le catalyseur (D) ainsi obtenu est constitué de 11 % en poids de UO₂MoO₄ déposés sur billes d'argile.

### EXEMPLE 5 - PREPARATION D'UN CATALYSEUR A BASE DE MOLYBDATE DE CALCIUM :

On prépare une solution (a) d'heptamolybdate d'ammonium par dissolution de 52,98 g de (NH₄)₆Mo₇O₂₄, 4H₂O (commercialisé par la Société MERCK) dans 200 cm3 d'eau permutée, et une solution (b) de nitrate de calcium par dissolution de 47,23 g de Ca(NO₃)₂,4H₂O (commercialisé par la Société PROLABO) dans 200 cm3 d'eau permutée. La solution (b) est ajoutée à la solution (a) dans un réacteur agité. Le pH du mélange, initialement de 4,5, est porté à 6,7 par ajout d'ammoniaque (commercialisée par la Société PROLABO). On chauffe sur plaque chauffante et la pâte obtenue est séchée à 120°C pendant 15 h environ ; le produit obtenu est ensuite broyé dans un mortier et calciné sous air à 500°C pendant 7 h.

Le produit (I) ainsi préparé a pour composition CaMoO₄, identifiée par diffraction des rayons X, et présente une surface spécifique mesurée selon la méthode B.E.T. de 5m2g⁻¹.

20 g du produit (I) sont saupoudrés lentement sur 123 g de support inerte composé de billes d'argile de diamètre moyen 4,8 mm, préalablement placées dans un drageoir en rotation et humidifiées par du glucose en solution aqueuse à 10 %. Dès que les billes sont sèches à l'extérieur, on pulvérise une petite quantité de la solution de glucose. Puis on saupoudre à nouveau le produit (I) sur les billes. On poursuit ces opérations alternativement jusqu'à enrobage de la totalité du produit (I). On sèche ensuite à 120°C pendant 2 h et calcine à 480°C pendant 6 h.

Le catalyseur (E) ainsi obtenu est constitué de 11 % en poids de CaMoO₄ déposés sur billes d'argile.

### EXEMPLE 6 - PREPARATION D'UN CATALYSEUR A BASE D'OXYDE MIXTE DE MOLYBDENE ET DE LANTHANE -

On prépare une solution (a) d'heptamolybdate d'ammonium par dissolution de 35,32 g de (NH₄)₆Mo₇O₂₄,4H₂O (commercialisé par la Société MERCK) dans 200 cm3 d'eau permutée, et une solution (b) de nitrate de lanthane par dilution de 71,64 cm3 de La(NO₃)₃ à 455 g.l⁻¹ en La₂O₃ (Provenance : Usine RHONE-POULENC de LA ROCHELLE) dans 200 cm3 d'eau permutée. La solution (a) est ajoutée à la solution (b) dans un réacteur agité. Le mélange est acidifié à pH=1-2 par ajout d'acide nitrique concentré. On chauffe sur plaque chauffante à 100-110°C et la pâte obtenue est séchée à 120°C pendant 15 h environ ; le produit obtenu est ensuite broyé dans un mortier et calciné sous air à 500°C pendant 3 h.

Le produit (I) ainsi préparé est constitué d'un mélange d'oxydes de lanthane et de molybdène, identifié par diffraction des rayons X, et présente une surface spécifique mesurée selon la méthode B.E.T. de 6m²g⁻¹.

20 g du produit (I) sont saupoudrés lentement sur 123 g de support inerte composé de billes d'argile de diamètre moyen 4,8 mm, préalablement placées dans un drageoir en rotation et humidifiées par du glucose en solution aqueuse à 10 %. Dès que les billes sont sèches à l'extérieur, on pulvérise une petite quantité de la solution de glucose. Puis on saupoudre à nouveau le produit (I) sur les billes. On poursuit ces opérations alternativement jusqu'à enrobage de la totalité du produit (I). On sèche ensuite à 120°C pendant 2 h et calcine à 480°C pendant 6 h.

Le catalyseur (F) ainsi obtenu est constitué de 9 % en poids de LaMoOₓ déposés sur billes d'argile.

### EXEMPLE 7 - PREPARATION D'UN CATALYSEUR A BASE DE MOLYBDATE DE FER -

On prépare une phase active de composition Fe₂(MoO₄)₃ selon le protocole opératoire décrit par S. NASU & S. SHIMIZU, J. Catal. 104, 164-175 (1987).

On prépare une solution (a) d'heptamolybdate d'ammonium par dissolution de 52,98 g de (NH₄)₆Mo₇O₂₄,4H₂O (commercialisé par la société MERCK) dans 200 cm3 d'eau permutée, et une solution (b) de nitrate de fer par dissolution de 80,8 g de Fe(NO₃)₃, 9H₂O (commercialisé par la Société PROLABO) dans 200 cm3 d'eau permutée. La solution (b) est ajoutée à la solution (a) dans un réacteur agité. Le pH du mélange est ajusté à 7,5 par ajout d'ammoniaque (commercialisée par la Société PROLABO). On chauffe sur plaque chauffante et la pâte obtenue est séchée à 120°C pendant 15 h environ ; le produit obtenu est ensuite broyé dans un mortier et calciné sous air à 500°C pendant 1 h, rebroyé et recalciné sous air à 500°C pendant 1 h.

Le produit (I) ainsi préparé a pour composition Fe₂(MoO₄)₃, identifiée par diffraction des rayons X, et présente une surface spécifique mesurée selon la méthode B.E.T. de 3,9m2g⁻¹.

20 g du produit (I) sont saupoudrés lentement sur 123 g de support inerte composé de billes d'argile de diamètre moyen 4,8 mm, préalablement placées dans un drageoir en rotation et humidifiées par du glucose en solution aqueuse à 10 %. Dès que les billes sont sèches à l'extérieur, on pulvérise une petite quantité de la solution de glucose. Puis on saupoudre à nouveau le produit (I) sur les billes. On poursuit ces opérations alternativement jusqu'à enrobage de la totalité du produit (I). On sèche ensuite à 120°C pendant 2 h et calcine à 480°C pendant 6 h.

Le catalyseur (G) ainsi obtenu est constitué de 13 % en poids de Fe₂(MoO₄)₃ déposés sur billes d'argile.

### EXEMPLE 8 - PREPARATION D'UN CATALYSEUR A BASE D'OXYDE MIXTE DE MOLYBDENE ET D'ETAIN -

(Ce catalyseur n'entre pas dans le cadre de l'invention).

On prépare une solution (a) d'heptamolybdate d'ammonium par dissolution de 35,32 g de (NH₄)₆Mo₇O₂₄,4H₂O (commercialisé par la Société MERCK) dans 200 cm3 d'eau permutée, et une solution (b) de chlorure d'étain par dissolution de 45,13 g de SnCl₂,2H₂O (commercialisé par la Société PROLABO) dans 200 cm3 d'eau permutée, acidifiée par quelques gouttes d'acide chlorhydrique concentré. La solution (b) est ajoutée à la solution (a) dans un réacteur agité. On chauffe le mélange et ajoute de l'ammoniaque (commercialisée par la Société PROLABO) jusqu'à pH neutre. On filtre sur verre fritté et lave avec 100 cm3 d'eau permutée ; le produit obtenu est séché à 120°C pendant 15 h environ, broyé dans un mortier et calciné sous air à 500°C pendant 3 h.

Le produit (I) ainsi préparé a pour composition SnO₂-2MoO₃, identifiée par diffraction des rayons X, et présente une surface spécifique mesurée selon la méthode B.E.T. de 26 m2g⁻¹.

20 g du produit (I) sont saupoudrés lentement sur 123 g de support inerte composé de billes d'argile de diamètre moyen 4,8 mm, préalablement placées dans un drageoir en rotation et humidifiées par du glucose en solution aqueuse à 10 %. Dès que les billes sont sèches à l'extérieur, on pulvérise une petite quantité de la solution de glucose. Puis on saupoudre à nouveau le produit (I) sur les billes. On poursuit ces opérations alternativement jusqu'à enrobage de la totalité du produit (I). On sèche ensuite à 120°C pendant 2 h et calcine à 480°C pendant 6 h.

Le catalyseur (H) ainsi obtenu est constitué de 11 % en poids de SnO₂-2MoO₃ déposés sur billes d'argile.

### EXEMPLE 9 - PREPARATION D'UN CATALYSEUR A BASE DE MOLYBDATE DE MANGANESE ET D'ALUMINE (30-70 % pds)

On prépare une solution (a) par dissolution de 26.47 g de (NH₄)₆Mo₇O₂₄, 4H₂O dans 120 cm3 d'eau permutée, une solution (b) par dissolution de 37.65 g de Mn(NO₃)₂, 4H₂O dans 60 cm3 d'eau permutée et une suspension (c) de 75.2 g d'alumine dans 100 cm3 d'eau permutée. On ajoute la solution (a) à la suspension (c), puis on ajoute la solution (b). On agite pendant 2 h et évapore à sec. On sèche ensuite à 120°C et calcine 500°C pendant 4 h. On obtient ainsi un produit (I).

20 g du produit (I) sont saupoudrés lentement sur 123 g de support inerte composé de billes d'argile de diamètre moyen 4.8 mm, préalablement placées dans un drageoir en rotation et humidifiées par du glucose en solution aqueuse à 10 %. Dès que les billes sont sèches à l'extérieur, on pulvérise une petite quantité de la solution de glucose. Puis on saupoudre à nouveau le produit (I) sur les billes. On poursuit ces opérations alternativement jusqu'à enrobage de la totalité du produit (I).

On sèche ensuite à 120°C pendant 2 h et calcine à 480°C pendant 6 h. Le catalyseur (J) ainsi obtenu est constitué de 11 % en poids de MnMoO₄-Al₂O₃ (30-70 % pds) déposés sur billes d'argile.

### EXEMPLE 10 - PREPARATION D'UN CATALYSEUR A BASE DE MOLYBDATE DE MANGANESE ET DE SILICE -

On prépare une solution (a) par dissolution de 58.3 g de (NH₄)₂Mo₂O₇ et 8.2 g de MoO₃ dans 150 cm3 d'eau permutée à 50-60°C. On prépare une solution (b) par dissolution de 100.4 g de Mn(NO₃)₂, 4H₂O dans 100 cm3 d'eau permutée. On ajoute la solution (b) à la solution (a) à température ambiante.

On imprègne ensuite à sec 31.9 cm3 étendus à 40 cm3 de la solution obtenue, sur 50 g de billes de silice. On sèche ensuite à 120°C pendant 15 h environ et calcine à 500°C pendant 4 h.

Le catalyseur (K) ainsi obtenu est constitué de 14.6 % en poids de MnMoO₄ déposés sur silice.

### MODE OPERATOIRE GENERAL DES TESTS D'AMMOXYDATION -

L'échantillon de catalyseur est préalablement porté sur un banc de mesure à la température de 150°C sous balayage d'hélium pendant 10 mn, puis il est soumis à un flux gazeux dont la composition sera précisée pour chaque exemple et qui renferme du propane, de l'ammoniac, de l'oxygène, de la vapeur d'eau et de l'hélium.

La pression totale du mélange réactionnel comprise entre 1 et 6 bar, sera également précisée pour chaque exemple.

Le débit gazeux est défini de façon à avoir une vitesse volumique horaire (VVH) comprise entre 100 et 36000 h⁻¹, dont la valeur précise sera indiquée pour chaque exemple.

Le principe du test d'ammoxydation du propane est le suivant :
- On porte le catalyseur à une température T₁, par exemple 300°C, et après 30 mn de stabilisation à la température T₁ on détermine par chromatographie en phase gazeuse la composition du mélange en sortie de réacteur.
- On calcule les pourcentages de conversion et les sélectivités obtenus sur le catalyseur examiné à la température d'entrée T₁ par des relations du type :
   Conversion du propane = % propane transformé / % propane introduit Sélectivité en acrylonitrile = % propane transformé en acrylonitrile / % propane converti
- On porte ensuite le catalyseur de 300 à 550°C par incrément de 20°C et on détermine toutes les 40 mn les pourcentages de conversion et les sélectivités.

Dans les exemples ci-après, les conventions suivantes sont utilisées :
- TTC3H8 =: conversion du propane
- SACN =: sélectivité en acrylonitrile
- SACN+C3H6 =: sélectivité en acrylonitrile et propylène
- SCOX =: sélectivité en monoxyde et dioxyde de carbone.

### EXEMPLES 11 A 13 -

Mesure des performances des catalyseurs (A), (B) et (D).

Les conditions opératoires mises en oeuvre sont les suivantes :
. Vitesse volumique horaire = 1000 h⁻¹
. Pression totale = 1.3 bar
. Composition du mélange réactionnel :
   C₃H₈ = 11 %
   NH₃ = 7,5 %
   O₂ = 10 %
   H₂O = 25 %
   He = 46,5 %

Les résultats et les conditions particulières figurent au tableau I ci-après :

**TABLEAU I**

| EXEMPLES | CATALYSEUR | T°C | TTC3H8 (%) | SACN (%) | SACN+C3H6(%) | SCOX (%) |
|---|---|---|---|---|---|---|
| 11 | (A) | 470 | 6 | 10 | 73 | 0 |
| 12 | (B) | 470 | 2 | 1 | 98 | 0 |
| | | 510 | 5,5 | 19 | 77 | 0 |
| 13 | (D) | 470 | 3 | 15 | 44 | 0 |
| | | 510 | 5 | 14 | 71 | 0 |

### EXEMPLES 14 A 16 ; essai témoin a :

Mesure des performances des catalyseurs (E) à (H).

Les conditions opératoires mises en oeuvre sont les suivantes :
. Vitesse volumique horaire = 1000 h⁻¹
. Pression totale = 1.3 bar
. Composition du mélange réactionnel :
   C₃H₈ = 30 %
   NH₃ = 15 %
   O₂ = 15 %
   H₂O = 20 %
   He = 20 %

Les résultats et les conditions particulières figurent au tableau II ci-après :

**TABLEAU II**

| EXEMPLES | CATALYSEUR | T°C | TTC3H8 (%) | SACN (%) | SACN+C3H6(%) | SCOX (%) |
|---|---|---|---|---|---|---|
| 14 | (E) | 475 | 2 | 0 | 78 | 0 |
| | | 490 | 4 | 9,5 | 69 | 0 |
| | | 460 | 1 | 3 | 83 | 0 |
| 15 | (F) | 490 | 7 | 19 | 55 | 0 |
| | | 510 | 7,5 | 10 | 60 | 6 |
| | | 460 | 5 | 20 | 59 | 0 |
| 16 | (G) | 475 | 7 | 23 | 56 | 0 |
| | | 490 | 6 | 19 | 58 | 13 |
| | | 440 | 9 | 6 | 32 | 19 |
| a | (H) | 460 | 11 | 20 | 43 | 16 |
| | | 475 | 11 | 24 | 47 | 13 |

### EXEMPLES 17 A 19 -

Mesure des performances du catalyseur (A) sous différentes pressions partielles de propane.

Les conditions opératoires mises en oeuvre sont les suivantes :
. Température = 510°C
. Vitesse volumique horaire = 1000 h⁻¹
. Pression totale = 1.3 bar
. Composition du mélange réactionnel :
   C₃H₈ = 11 % ou 19 ou 26 %
   NH₃ = 7,5 %
   O₂ =10 %
   H₂O = 25 %
   He = 31,5 % ou 38,5 ou 46,5 %

Les résultats et les conditions particulières figurent au tableau III ci-après :

**TABLEAU III**

| EXEMPLES | C3H8 (%) | TTC3H8 (%) | SACN (%) | SACN+ C3H6(%) | SCOX (%) |
|---|---|---|---|---|---|
| 17 | 11 | 17 | 3 | 50 | 21 |
| 18 | 19 | 16 | 16 | 69 | 5 |
| 19 | 26 | 16 | 13 | 72 | 5,5 |

### EXEMPLES 20 A 23 -

Mesure des performances du catalyseur (B) sous différentes températures.

Les conditions opératoires mises en oeuvre sont les suivante
. Vitesse volumique horaire = 1000 h⁻¹
. Pression totale = 1.3 bar
. Composition du mélange réactionnel :
   C₃H₈ = 19 %
   NH₃ = 7,5 %
   O₂ =10 %
   H₂O = 25 %
   He = 38,5 %

Les résultats et les conditions particulières figurent au tableau IV ci-après :

**TABLEAU IV**

| EXEMPLES | T° C | TTC3H8 (%) | SACN (%) | SACN+C3H6(%) | SCOX (%) |
|---|---|---|---|---|---|
| 20 | 420 | 0,4 | 16 | 81 | 0 |
| 21 | 470 | 2 | 17 | 72 | 0 |
| 22 | 485 | 3 | 17 | 71 | 0 |
| 23 | 510 | 8 | 30 | 67 | 0 |

### EXEMPLE 24 A 26 :

Mesure des performances du catalyseur (B) à différentes vitesses volumiques horaires.

Les conditions opératoires mises en oeuvre sont les suivantes :
. Température = 510°C
. Pression totale = 1.3 bar
. Composition du mélange réactionnel :
   C₃H₈ = 19 %
   NH₃ = 7,5 %
   O₂ =10 %
   H₂O = 25 %
   He = 38,5 %

Les conditions particulières ainsi que les résultats obtenus figurent au tableau V ci-après :

**TABLEAU V**

| EXEMPLES | VVH(h-1) | Volcata (ml) | TTC3H8 (%) | SACN (%) | SACN+C3H6(%) | SCOX (%) |
|---|---|---|---|---|---|---|
| 24 | 200 | 100 | 13 | 21 | 77 | 2 |
| 25 | 500 | 40 | 13 | 27 | 65 | 0 |
| 26 | 1 000 | 20 | 8 | 30 | 67 | 0 |

### EXEMPLES 27 A 31 -

Mesure des performances du catalyseur (B) sous différentes températures.

Les conditions opératoires mises en oeuvre sont les suivantes :
. Vitesse volumique horaire = 500 h⁻¹
. Pression totale = 1.3 bar
. Composition du mélange réactionnel :
   C₃H₈ = 19 %
   NH₃ = 7,5 %
   O₂ =10 %
   H₂O = 25 %
   He = 38,5 %

Les résultats et les conditions particulières figurent au tableau VI ci-après :

**TABLEAU VI**

| EXEMPLES | T° C | TTC3H8 (%) | SACN (%) | SACN+ C3H6(%) | SCOX (%) |
|---|---|---|---|---|---|
| 27 | 420 | 1 | 3 | 87 | 0 |
| 28 | 450 | 3 | 15 | 58 | 0 |
| 29 | 470 | 5 | 20 | 62 | 0 |
| 30 | 490 | 9 | 27 | 62 | 0 |
| 31 | 510 | 13 | 27 | 65 | 0 |

### EXEMPLES 32 A 36 :

Mesure des performances du catalyseur (C) à différentes températures.

Les conditions opératoires mises en oeuvre sont les suivantes :
. Vitesse volumique horaire = 1000h⁻¹
. Pression totale = 1.3 bar
. Composition du mélange réactionnel :
   C₃H₈ = 19 %
   NH₃ = 7,5 %
   O₂ =10 %
   H₂O = 25 %
   He = 38,5 %

Les conditions particulières ainsi que les résultats obtenus figurent au tableau VII ci-après :

**TABLEAU VII**

| EXEMPLES | T° C | TTC3H8 (%) | SACN (%) | SACN+C3H6(%) | SCOX (%) |
|---|---|---|---|---|---|
| 32 | 410 | 3 | 9 | 64 | 0 |
| 33 | 430 | 6 | 13 | 74 | 0 |
| 34 | 470 | 11 | 16 | 76 | 4 |
| 35 | 490 | 20 | 23 | 73 | 9 |
| 36 | 510 | 24 | 24 | 73 | 6 |

### EXEMPLES 37 A 39 :

Mesure des performances du catalyseur (B) sous différentes pressions partielles de propane.

Les conditions opératoires mises en oeuvre sont les suivantes :
. Température = 510°C
. Vitesse volumique horaire = 1000h⁻¹
. Pression totale = 1.3 bar
. Composition du mélange réactionnel :
   C₃H₈ = 11% ou 19 ou 26 %
   NH₃ = 7,5 %
   O₂ =10 %
   H₂O = 25 %
   He = 31,5 % ou 38,5 ou 46,5 %

Les conditions particulières ainsi que les résultats obtenus figurent au tableau VIII ci-après :

**TABLEAU VIII**

| EXEMPLES | C3H8 (%) | TTC3H8 (%) | SACN(%) | SACN+C3H6(%) | SCOX (%) |
|---|---|---|---|---|---|
| 37 | 11 | 5 | 19 | 77 | 0 |
| 38 | 19 | 8 | 30 | 67 | 0 |
| 39 | 26 | 5 | 13 | 80 | 0 |

### EXEMPLES 40 A 42 :

Mesure des performances du catalyseur (B) sous différentes pressions partielles d'ammoniac.

Les conditions opératoires mises en oeuvre sont les suivantes :
. Température = 510°C
. Vitesse volumique horaire = 1000h⁻¹
. Pression totale = 1.3 bar
. Composition du mélange réactionnel :
   C₃H₈ = 19 %
   NH₃ = 4 % ou 7,5 ou 11 %
   O₂ =10 %
   H₂O = 25 %
   He = 35 % ou 38,5 ou 42 %

Les conditions particulières ainsi que les résultats obtenus figurent au tableau IX ci-après :

**TABLEAU IX**

| EXEMPLES | NH3 (%) | TTC3H8 (%) | SACN (%) | SACN+C3H6(%) | SCOX (%) |
|---|---|---|---|---|---|
| 40 | 4 | 4 | 0 | 82 | 17 |
| 41 | 7,5 | 8 | 30 | 67 | 0 |
| 42 | 11 | 10 | 30 | 60 | 0 |

### EXEMPLES 43 A 45 :

Mesure des performances du catalyseur (D) sous différentes pressions partielles de propane.

Les conditions opératoires mises en oeuvre sont les suivantes :
. Température = 510°C
. Vitesse volumique horaire = 1000h⁻¹
. Pression totale = 1.3 bar
. Composition du mélange réactionnel :
   C₃H₈ = 11 % ou 19 ou 26 %
   NH₃ = 7,5 %
   O₂ =10 %
   H₂O = 25 %
   He = 31,5 % ou 38,5 ou 46,5 %

Les conditions particulières ainsi que les résultats obtenus figurent au tableau X ci-après :

**TABLEAU X**

| EXEMPLES | C3H8 (%) | TTC3H8 (%) | SACN (%) | SACN+C3H6(%) | SCOX (%) |
|---|---|---|---|---|---|
| 43 | 11 | 5 | 14 | 71 | 0 |
| 44 | 19 | 4 | 11 | 73 | 0 |
| 45 | 26 | 7 | 22 | 54 | 0 |

### EXEMPLES 46 A 48 :

Mesure des performances du catalyseur (D) sous différentes pressions partielles d'ammoniac.

Les conditions opératoires mises en oeuvre sont les suivantes :
. Température = 510°C
. Vitesse volumique horaire = 1000h⁻¹
. Pression totale = 1.3 bar
. Composition du mélange réactionnel :
   C₃H₈ = 19 %
   NH₃ = 4 % ou 7,5 ou 11 %
   O₂ =10 %
   H₂O = 25 %
   He = 35 % ou 38,5 ou 42 %

Les conditions particulières ainsi que les résultats obtenus figurent au tableau XI ci-après :

**TABLEAU XI**

| EXEMPLES | NH3 (%) | TTC3H8 (%) | SACN (%) | SACN+C3H6(%) | SCOX (%) |
|---|---|---|---|---|---|
| 46 | 4 | 6 | 24 | 62 | 0 |
| 47 | 7,5 | 4 | 11 | 73 | 0 |
| 48 | 11 | 4 | 13 | 70 | 0 |

### EXEMPLE 49 A 51 :

Mesure des performances du catalyseur (F) sous différentes pressions partielles d'oxygène.

Les conditions opératoires mises en oeuvre sont les suivantes :
. Température = 490°C
. Vitesse volumique horaire = 1000h⁻¹
. Pression totale = 1.3 bar
. Composition du mélange réactionnel :
   C₃H₈ = 20 %
   NH₃ = 15 %
   O₂ = 5 % ou 15 ou 25 %
   H₂O = 20 %
   He = 20 % ou 30 ou 40 %

Les conditions particulières ainsi que les résultats obtenus figurent au tableau XII ci-après :

**TABLEAU XII**

| EXEMPLES | O2 (%) | TTC3H8 (%) | SACN (%) | SACN+C3H6(%) | SCOX (%) |
|---|---|---|---|---|---|
| 49 | 5 | 4 | 15 | 63 | 0 |
| 50 | 15 | 6 | 14 | 59 | 0 |
| 51 | 25 | 8 | 18 | 58 | 4 |

### EXEMPLES 52 ET 53 :

Mesure des performances du catalyseur (G) sous différentes pressions partielles d'ammoniac.

Les conditions opératoires mises en oeuvre sont les suivantes :
. Température = 490°C
. Vitesse volumique horaire = 1000h⁻¹
. Pression totale = 1.3 bar
. Composition du mélange réactionnel :
   C₃H₈ = 20 %
   NH₃ = 5 % ou 25 %
   O₂ = 15 %
   H₂O = 20 %
   He = 20 % ou 40 %

Les conditions particulières ainsi que les résultats obtenus figurent au tableau XIII ci-après :

**TABLEAU XIII**

| EXEMPLES | NH3 (%) | TTC3H8 (%) | SACN (%) | SACN+C3H6(%) | SCOX (%) |
|---|---|---|---|---|---|
| 52 | 5 | 12 | 17 | 45 | 28 |
| 53 | 25 | 14 | 7 | 42 | 4 |

### EXEMPLE 54 et 55 :

Mesure des performances du catalyseur (G) sous différentes pressions partielles d'oxygène.

Les conditions opératoires mises en oeuvre sont les suivantes :
. Température = 475°C
. Vitesse volumique horaire = 1000h⁻¹
. Pression totale = 1.3 bar
. Composition du mélange réactionnel :
   C₃H₈ = 20 %
   NH₃ = 15 %
   O₂ = 5 % ou 25 %
   H₂O = 20 %
   He = 20 % ou 40 %

Les conditions particulières ainsi que les résultats obtenus figurent au tableau XIV ci-après :

**TABLEAU XIV**

| EXEMPLES | O2 (%) | TTC3H8 (%) | SACN (%) | SACN+C3H6(%) | SCOX (%) |
|---|---|---|---|---|---|
| 54 | 5 | 8 | 15 | 58 | 4 |
| 55 | 25 | 11 | 17 | 60 | 14 |

### EXEMPLES 56 ET 57 :

Mesure des performances des catalyseurs (J) et (K).

Les conditions opératoires mises en oeuvre sont les suivantes :
. Vitesse volumique horaire = 1000 h⁻¹
. Pression totale = 1.3 bar
. Composition du mélange réactionnel :
   C₃H₈ = 48 %
   NH₃ = 9 %
   O₂ = 18 %
   H₂O = 20 %
   He = 5 %

Les conditions particulières ainsi que les résultats obtenus figurent au tableau XV ci-après :

**TABLEAU XV**

| EXEMPLES | CATALYSEUR | T°C | TTC3H8 (%) | SACN (%) | SACN+C3H6(%) | SCOX (%) |
|---|---|---|---|---|---|---|
| 56 | (J) | 500 | 15 | 15 | 62 | 0 |
| 57 | (K) | 460 | 12 | 14 | 37 | 0,2 |

### Essais témoins b, c, d :

Mesure des performances du catalyseur (H) à différentes températures.

Les conditions opératoires mises en oeuvre sont les suivantes :
. Vitesse volumique horaire = 1000h⁻¹
. Pression totale = 1.3 bar
. Composition du mélange réactionnel :
   C₃H₈ = 20 %
   NH₃ = 5 %
   O₂ = 15 %
   H₂O = 20 %
   He = 40 %

Les conditions particulières ainsi que les résultats obtenus figurent au tableau XVI ci-après :

**TABLEAU XVI**

| EXEMPLES | T°C | TTC3H8 (%) | SACN (%) | SACN+C3H6(%) | SCOX (%) |
|---|---|---|---|---|---|
| b | 460 | 19 | 13 | 25 | 38 |
| c | 475 | 20 | 19 | 32 | 43 |
| d | 490 | 21 | 26 | 41 | 28 |

### EXEMPLE 58 - PREPARATION D'UN CATALYSEUR A BASE DE MOLYBDATE D'URANYLE

On prépare une phase active de composition UMo₁₀0x selon le protocole opératoire suivant.

On prépare une solution (a) d'heptamolybdate d'ammonium par dissolution de 176,56 g de (NH₄)₆Mo₇O₂₄,4H₂O (commercialisé par la Société MERCK) dans 400 cm3 d'eau permutée, et une solution (b) de nitrate d'uranyle par dissolution de 50,2 g de U0₂(NO₃)₂, 6H₂O (commercialisé par la Société PROLABO) dans 50 cm3 d'eau permutée. La solution (b) est ajoutée à la solution (a) dans un réacteur agité et on chauffe à 80°C environ. On maintient à 80 - 100°C pendant 2 heures environ et la pâte obtenue est séchée à 120°C pendant 15 h environ ; le produit obtenu est ensuite calciné sous air à 500°C pendant 4 h.

Le produit ainsi préparé présente une surface spécifique mesurée selon la méthode B.E.T. de 2,4 m2g⁻¹.

10 g de ce produit sont saupoudrés lentement sur 65 g de support inerte composé de billes d'argile de diamètre moyen 4,8 mm, préalablement placées dans un drageoir en rotation et humidifiées par du glucose en solution aqueuse à 10 %. Dès que les billes sont sèches à l'extérieur, on pulvérise une petite quantité de la solution de glucose. Puis on saupoudre à nouveau le produit sur les billes. On poursuit ces opérations alternativement jusqu'à enrobage de la totalité du produit. On sèche ensuite à 120°C pendant 2 h et calcine à 480°C pendant 6 h.

Le catalyseur (L) ainsi obtenu est constitué de 11,2 % en poids de UMo₁₀Ox déposés sur billes d'argile.

### EXEMPLE 59 - PREPARATION D'UN CATALYSEUR A BASE DE MOLYBDATE DE COBALT

On prépare une phase active de composition CoMoO₄ selon le protocole opératoire suivant.

On prépare une solution (a) d'heptamolybdate d'ammonium par dissolution de 70,6 g de (NH₄)₆Mo₇O₂₄,4H₂O (commercialisé par la Société MERCK) dans 400 cm3 d'eau permutée, et une solution (b) de nitrate de cobalt par dissolution de 116,4 g de Co(NO₃)₂, 6H₂O dans 150 cm3 d'eau permutée. La solution (b) est ajoutée à la solution (a) dans un réacteur agité et on chauffe à ébullition. La pâte obtenue est séchée à 120°C pendant 15 h environ, puis est calcinée sous air à 500°C pendant 4 h.

Le produit ainsi préparé présente une surface spécifique mesurée selon la méthode B.E.T. de 7,7 m2g⁻¹.

10 g de ce produit sont saupoudrés lentement sur 50 g de support inerte composé de billes d'argile de diamètre moyen 4,8 mm, préalablement placées dans un drageoir en rotation et humidifiées par du glucose en solution aqueuse à 10 %. Dès que les billes sont sèches à l'extérieur, on pulvérise une petite quantité de la solution de glucose. Puis on saupoudre à nouveau le produit sur les billes. On poursuit ces opérations alternativement jusqu'à enrobage de la totalité du produit. On sèche ensuite à 120°C pendant 2 h et calcine à 480°C pendant 6 h.

Le catalyseur (M) ainsi obtenu est constitué de 11,7 % en poids de CoMoO₄ déposés sur billes d'argile.

### EXEMPLE 60 - PREPARATION D'UN CATALYSEUR A BASE DE MOLYBDATE DE COBALT

On prépare une phase active de composition CoMoO₄ selon le protocole opératoire suivant.

On prépare une solution (a) d'heptamolybdate d'ammonium par dissolution de 70,6 g de (NH₄)₆Mo₇O₂₄,4H₂O (commercialisé par la Société MERCK) dans 400 cm3 d'eau permutée, et une solution (b) de nitrate de cobalt par dissolution de 116,4 g de Co(NO₃)₂, 6H₂O dans 150 cm3 d'eau permutée. La solution (b) est ajoutée à la solution (a) dans un réacteur agité et on chauffe à ébullition. La pâte obtenue est filtrée sur verre fritté, est séchée à 120°C pendant 15 h environ, puis est calcinée sous air à 500°C pendant 4 h.

Le produit ainsi préparé présente une surface spécifique mesurée selon la méthode B.E.T. de 13 m2g⁻¹.

10 g de ce produit sont saupoudrés lentement sur 67 g de support inerte composé de billes d'argile de diamètre moyen 4,8 mm, préalablement placées dans un drageoir en rotation et humidifiées par du glucose en solution aqueuse à 10 %. Dès que les billes sont sèches à l'extérieur, on pulvérise une petite quantité de la solution de glucose. Puis on saupoudre à nouveau le produit sur les billes. On poursuit ces opérations alternativement jusqu'à enrobage de la totalité du produit. On sèche ensuite à 120°C pendant 2 h et calcine à 480°C pendant 6 h.

Le catalyseur (N) ainsi obtenu est constitué de 10 % en poids de CoMoO₄ déposés sur billes d'argile.

### EXEMPLES 61 A 65 :

Mesure des performances du catalyseur (L) à différentes températures et pour différentes compositions du mélange réactionnel.

Les conditions opératoires mises en oeuvre sont les suivantes :
. Vitesse volumique horaire = 1000 h⁻¹
. Pression totale = 1.3 bar

La composition du mélange réactionnel, la température ainsi que les résultats obtenus figurent dans le tableau XVII ci-après.

**TABLE XVII**

| | | | | | |
|---|---|---|---|---|---|
| EXEMPLES | 61 | 62 | 63 | 64 | 65 |
| C₃H₈ (% vol) | 7.5 | 25 | 25 | 40 | 48 |
| NH₃ (% vol) | 15 | 25 | 10 | 15 | 9 |
| O₂ (% vol) | 15 | 10 | 25 | 15 | 18 |
| He (% vol) | 42.5 | 20 | 20 | 10 | 5 |
| T (°C) | 460 | 430 | 460 | 460 | 480 |
| TTC₃H₈ (%) | 4 | 2 | 5 | 3 | 6 |
| SACN (%) | 19 | 15 | 13 | 13 | 18 |
| SACN + C₃H₆ (%) | 48 | 59 | 32 | 78 | 44 |
| SCOx (%) | 0 | 0 | 0 | 1 | 0 |

### EXEMPLES 66 A 69 :

Mesure des performances du catalyseur (M) à différentes températures et pour différentes compositions du mélange réactionnel.

Les conditions opératoires mises en oeuvre sont les suivantes :
. Vitesse volumique horaire = 1000 h⁻¹
. Pression totale = 1.3 bar

La composition du mélange réactionnel, la température ainsi que les résultats obtenus figurent dans le tableau XVIII ci-après.

**TABLEAU XVIII**

| | | | | | | | | | |
|---|---|---|---|---|---|---|---|---|---|
| EXEMPLES | 66 | | 67 | | 68 | | 69 | | |
| C₃H₈ (% vol) | 7.5 | | 25 | | 40 | | 48 | | |
| NH₃ (% vol) | 15 | | 25 | | 15 | | 9 | | |
| O₂ (% vol) | 15 | | 10 | | 15 | | 18 | | |
| He (% vol) | 42.5 | | 20 | | 10 | | 5 | | |
| T (°C) | 460 | 480 | 460 | 480 | 460 | 480 | 440 | 460 | 480 |
| TTC₃H₈ (%) | 8 | 17 | 6 | 6 | 7 | 11 | 6 | 10 | 16 |
| SACN (%) | 20 | 31 | 14 | 12 | 13 | 17 | 16 | 18 | 17 |
| SACN + C₃H₆ (%) | 76 | 75 | 59 | 67 | 83 | 84 | 73 | 79 | 75 |
| SCOx (%) | 0 | 0 | 0 | 0 | 0 | 1 | 0 | 1 | 5 |

### EXEMPLES 70 A 73 :

Mesure des performances du catalyseur (N) à différentes températures et pour différentes compositions du mélange réactionnel.

Les conditions opératoires mises en oeuvre sont les suivantes :
. Vitesse volumique horaire = 1000 h⁻¹
. Pression totale = 1.3 bar

La composition du mélange réactionnel, la température ainsi que les résultats obtenus figurent dans le tableau XIX ci-après.

**TABLEAU XIX**

| | | | | | | |
|---|---|---|---|---|---|---|
| EXEMPLES | 70 | 71 | | 72 | | 73 |
| C₃H₈ (% vol) | 7.5 | 25 | | 40 | | 48 |
| NH₃ (% vol) | 15 | 10 | | 15 | | 9 |
| O₂ (% vol) | 15 | 25 | | 15 | | 18 |
| He (% vol) | 42.5 | 20 | | 10 | | 5 |
| T (°C) | 480 | 460 | 480 | 440 | 460 | 440 |
| TTC₃H₈ (%) | 11 | 8 | 15 | 6 | 11 | 5 |
| SACN (%) | 14 | 17 | 20 | 11 | 17 | 12 |
| SACN + C₃H₆ (%) | 84 | 82 | 76 | 83 | 83 | 82 |
| SCOx (%) | 2 | 0 | 4 | 0 | 1 | 0 |

## Revendications

1. Procédé d'ammoxydation d'alcanes en phase vapeur en présence d'un catalyseur solide dont la phase active renferme du molybdène et de l'oxygène caractérisé en ce que la dite phase active est un oxyde mixte à base de molybdène ou un orthomolybdate qui renferme également au moins un élément choisi dans le groupe constitué par les métaux alcalino-terreux, le manganèse, le fer, le cobalt, l'uranium et le lanthane.

2. Procédé selon la revendication 1, caractérisé en ce que ladite phase active renferme au maximum 2 éléments choisis dans le groupe défini ci-avant.

3. Procédé selon la revendication 1, caractérisé en ce que ladite phase active est un molybdate de manganèse, d'uranyle, de cobalt ou de fer.

4. Procédé selon l'une quelconque des revendications précédentes, caractérisé en ce que l'alcane est le propane.

5. Procédé selon l'une quelconque des revendications précédentes, caractérisé en ce que la réaction est conduite en présence de vapeur d'eau.

6. Procédé selon l'une quelconque des revendications précédentes, caractérisé en ce que la température de réaction est comprise entre 350°C et 550°C.

7. Procédé selon l'une quelconque des revendications précédentes, caractérisé en ce que la pression totale est comprise entre 1 et 6 bar.

8. Procédé selon l'une quelconque des revendications précédentes, caractérisé en ce que la vitesse volumique horaire est comprise entre 100 et 36 000 h⁻¹.

9. Procédé selon l'une quelconque des revendications précédentes, caractérisé en ce que dans le gaz réactif (mélange d'hydrocarbure saturé, d'ammoniac et d'oxygène) la teneur en hydrocarbure saturé est comprise entre 5 et 70 %, la teneur en ammoniac est comprise entre 3 et 50 % et la teneur en oxygène est comprise entre 3 et 45 %.

10. Procédé selon l'une quelconque des revendications précédentes, caractérisé en ce que la composition du mélange gazeux est en dehors du domaine d'explosivité.

11. Procédé selon l'une quelconque des revendications précédentes, caractérisé en ce que le catalyseur solide renferme, outre ladite phase active, un support.

## Patentansprüche

1. Verfahren zur Ammonoxidation von Alkanen in der Dampfphase in Gegenwart eines festen Katalysators, dessen aktive Phase Molybdän und Sauerstoff enthält, dadurch gekennzeichnet, daß die genannte aktive Phase ein Mischoxid auf der Basis von Molybdän oder einem Orthomolybdat ist, die auch wenigstens ein aus der Gruppe, die aus den Erdalkalimetallen, Mangan, Eisen, Kobalt, Uran und Lanthan besteht, ausgewähltes Element enthält.

2. Verfahren gemäß Anspruch 1, dadurch gekennzeichnet, daß besagte aktive Phase höchstens 2 Elemente aus der vorstehend definierten Gruppe einschließt.

3. Verfahren gemäß Anspruch 1, dadurch gekennzeichnet, daß besagte aktive Phase ein Mangan-, Uranyl-, Kobalt- oder Eisenmolybdat ist.

4. Verfahren gemäß einem der vorhergehenden Ansprüche, dadurch gekennzeichnet, daß das Alkan Propan ist.

5. Verfahren gemäß einem der vorhergehenden Ansprüche, dadurch gekennzeichnet, daß die Reaktion in Gegenwart von Wasserdampf durchgeführt wird.

6. Verfahren gemäß einem der vorhergehenden Ansprüche, dadurch gekennzeichnet, daß die Reaktionstemperatur zwischen 350 °C und 550 °C liegt.

7. Verfahren gemäß einem der vorhergehenden Ansprüche, dadurch gekennzeichnet, daß der Gesamtdruck zwischen 1 und 6 bar liegt.

8. Verfahren gemäß einem der vorhergehenden Ansprüche, dadurch gekennzeichnet, daß die Volumengeschwindigkeit pro Stunde zwischen 100 und 36 000 h⁻¹ liegt.

9. Verfahren gemäß einem der vorhergehenden Ansprüche, dadurch gekennzeichnet, daß in dem Reaktivgas (Gemisch aus gesättigtem Kohlenwasserstoff, Ammoniak und Sauerstoff) der Gehalt an gesättigtem Kohlenwasserstoff zwischen 5 und 70% liegt, der Ammoniak-Gehalt zwischen 3 und 50% liegt und der Sauerstoff-Gehalt zwischen 3 und 45% liegt.

10. Verfahren gemäß einem der vorhergehenden Ansprüche, dadurch gekennzeichnet, daß die Zusammensetzung des Gasgemisches außerhalb des Explosionsbereichs liegt.

11. Verfahren gemäß einem der vorhergehenden Ansprüche, dadurch gekennzeichnet, daß der feste Katalysator außer besagter aktiver Phase einen Träger einschließt.

## Claims

1. Process for ammoxidation of alkanes in vapour phase in the presence of a solid catalyst whose active phase contains molybdenum and oxygen, characterised in that the said active phase is a mixed oxide based on molybdenum or is an orthomolybdate which contains at least one element chosen from the group consisting of the alkaline-earth metals, manganese, iron, cobalt, uranium and lanthanum.

2. Process according to claim 1, characterised in that the said active phase contains at most 2 elements chosen from the group defined above.

3. Process according to claim 1, characterised in that the said active phase is a manganese, uranyl, cobalt or iron molybdate.

4. Process according to any one of the preceding claims, characterised in that the alkane is propane.

5. Process according to any one of the preceding claims, characterised in that the reaction is conducted in the presence of water vapour.

6. Process according to any one of the preceding claims, characterised in that the reaction temperature is between 350°C and 550°C.

7. Process according to any one of the preceding claims, characterised in that the total pressure is between 1 and 6 bar.

8. Process according to any one of the preceding claims, characterised in that the hourly space velocity is between 100 and 36,000 h⁻¹.

9. Process according to any one of the preceding claims, characterised in that in the reactant gas (mixture of saturated hydrocarbon, ammonia and oxygen) the saturated hydrocarbon content is between 5 and 70 %, the ammonia content is between 3 and 50 % and the oxygen content is between 3 and 45 %.

10. Process according to any one of the preceding claims, characterised in that the composition of the gaseous mixture is outside the explosive region.

11. Process according to any one of the preceding claims, characterised in that, in addition to the said active phase, the solid catalyst contains a support.
